Europäisches Patentamt

European Patent Office

Office européen des brevets

(11) Publication number: 0 380 374
A1

# (12) EUROPEAN PATENT APPLICATION

(21) Application number: 90300906.6

(22) Date of filing: 29.01.90

(51) Int. Cl.5: **C07C 5/25, C07C 7/148, C07C 11/08**

(30) Priority: 27.01.89 JP 18350/89

(43) Date of publication of application:
01.08.90 Bulletin 90/31

(84) Designated Contracting States:
BE DE ES FR GB IT NL

(71) Applicant: **TONEN SEKIYU KAGAKU KABUSHIKI KAISHA**
**1-1, Tsukiji, 4-chome**
**Chuo-ku Tokyo(JP)**

(72) Inventor: **Kohara, Tadanao**
**8-116, Shimoda-cho 5-chome, Kohoku-ku**
**Yokohama-shi, Kanagawa-ken(JP)**
Inventor: **Hara, Yasuji**
**10-2, Shiomidai 3-chome**
**Yokosuki-shi, Kanagawa-ken(JP)**
Inventor: **Okada, Yukiko**
**1-8, Kitakoiwa 4-chome, Edogawa-ku**
**Tokyo(JP)**
Inventor: **Kamiyama, Setsuo**
**119-7, Yamada**
**Kawagoe-shi, Saitama-ken(JP)**

(74) Representative: **Northover, Robert Frank et al**
**Exxon Chemical Limited Exxon Chemical**
**Technology Centre PO Box 1**
**Abingdon Oxfordshire, OX13 6BB(GB)**

(54) Method of isomerizing butene-1 contained in isobutylene into butene-2.

(57) A method of isomerizing butene-1 which is present in a isobutylene feed into butene-2. The method is carried out by contacting the isobutylene feed with a catalyst containing a Group VIII metal in the presence of hydrogen and carbon monoxide.

EP 0 380 374 A1

## METHOD OF ISOMERIZING BUTENE-1 CONTAINED IN ISOBUTYLENE INTO BUTENE-2

BACKGROUND OF THE INVENTION

The present invention relates to a method of isomerizing butene-1 contained in an isobutylene feed into butene-2 and, more particularly, relates to a process for producing isobutylene having high purity which is suitable for producing butyl rubber with reduced butene-1 content by isomerizing butene-1 contained in an isobutylene feed into butene-2 and removing the resultant butene-2 by distillation.

Since butene-1 which is present as an impurity in isobutylene used to produce butyl rubber inhibits the polymerizing of isobutylene, it is necessary to apply strict control of the butene-1 concentration in isobutylene to less than a predetermined concentration. However, since the boiling points of butene-1 and isobutylene are close to one another (0.6 °C) separating butene-1 from isobutylene by distillation is extremely difficult. Thus, it is only possible to obtain isobutylene of high purity in which the butene-1 content is reduced to less than a predetermined concentration by at first isomerizing butene-1 into butene-2 which has a significant difference of the boiling point with respect to isobutylene and then removing the resultant butene-2 by distillation.

Accordingly, there is a need for a method of isomerizing butene-1 into butene-2 capable of selectively isomerizing butene-1 contained in an isobutylene feed into butene-2, with extremely less isobutylene loss upon isomerizing reaction.

The following methods of isomerizing butene-1 into butene-2 have been disclosed.

(a) A method of isomerizing a hydrocarbon feed containing butene-1 in a liquid phase under the presence of hydrogen using a palladium catalyst applied with sulfur treatment (refer to Japanese Patent Publications Sho 47-48362 and Sho 57-40812, Japanese Patent Laid-Open Sho 59-20232, and Japanese Patent Publication Sho 60-54934).

However, the above method has an aim of collecting the butene-2 fraction by isomerizing butene-1 present in a great amount in a $C_4$ hydrocarbon feed and thus, the method is especially adapted for a $C_4$ hydrocarbon feed containing butene-1 at a high concentration, such as an amount greater than 10 % of butene-1 content in the $C_4$ hydrocarbon feed. The present invention is directed to isobutylene containing a small amount of butene-1. In addition, when a isobutylene feed containing from 0.2 to 2.0 % by weight of butene-1 as in the present invention is processed by using a palladium catalyst applied with a sulfur treatment, although isomerization from butene-1 to butene-2 proceeds, a slight amount of a sulfur compound is mixed into the isobutylene feed which results in requiring a facility for removing the sulfur compound from the isobutylene feed in order to use the product as isobutylene for producing butyl rubber.

(b) A method of isomerizing butene-1 contained in a hydrocarbon feed in a liquid or gas phase at a low temperature under the presence of hydrogen using a palladium catalyst (Japanese Patent Laid-Open Sho 48-602).

In the above method, a hydrocarbon feed containing $C_3$ or higher 1-olefins is isomerized within a temperature range of 0 to -70 °C. A problem of the method is that a refrigeration facility is required for keeping the reaction at a low temperature and a great energy is consumed upon reaction.

(c) A method of isomerizing a small amount of butene-1 contained in isobutylene in a gas phase under the presence of hydrogen using a palladium catalyst (Japanese Patent Laid-Open Sho 59-5129).

In the above method, from 0.06 to 0.10 % by weight of butene-1 in isobutylene is isomerized under a gas phase. However, when the isomerizing reaction for 0.2 to 2.0 % by weight of butene-1 contained in isobutylene feed is carried out in the gas phase, the catalyst activity is reduced sooner when compared with a liquid phase-eaction and, in addition, since the permissible range for the molar ratio of hydrogen relative to isobutylene feed is narrow, the range for operation is narrow.

(d) The present applicants have also proposed an isomerizing method by bringing from 0.2 to 2.0 % by weight of butene-1 contained in isobutylene into contact with a catalyst containing a group VIII metal in a liquid phase under the presence of hydrogen (Japanese Patent Application Sho 62-217072).

In addition, the following methods have been prepared for selectively hydrogenating acetylenes and diolefins in a hydrocarbon feed using hydrogen containing carbon monoxide and a method of applying a carbon monoxide pre-treatment to the catalyst which is used for the isomerizing reaction of butene-1 to butene-2.

(e) A method of selectively hydrogenating acetylenes and diolefins contained in a $C_4$ hydrocarbon feed with hydrogen containing carbon monoxide (Japanese Patent Laid-Open Sho 61-18731, Japanese Patent Publication Sho 63-25566, etc.).

When acetylenes and diolefins are hydrogenated by the method disclosed above, acetylenes and diolefins contained are selectively hydrogenated to produce corresponding monoolefins. However, butene-1 is not isomerized into butene-2. That is, it has been reported in the patent publications that the isomerizing reaction of butene-1 into butene-2 is restricted under the presence of carbon monoxide.

(f) A method of isomerizing butene-1 in a hydrocarbon feed into butene-2 by improving the isomerizing performance of a catalyst comprising group VIII metal after inactivating the same by contact with carbon monoxide and then re-activating with hydrogen (U.S. Patent 3,764,633).

However, when the present inventors conducted a pre-treatment of the group VIII metal catalyst with carbon monoxide by the method and used the catalyst for the isomerizing reaction as disclosed above, it has been found that the isomerizing performance has not been appreciably improved.

Therefore, there is a need for a method of improving the isomerizing performance of butene-1 into butene-2 by using hydrogen containing carbon monoxide while suppressing hydrogenation as the side reaction.

## SUMMARY OF THE INVENTION

The object of the invention is to mitigate certain problems in the prior art when isomerizing butene-1 contained in an isobutylene feed into butene-2 in that a slight amount of unfavorable ingredient such as sulfur is leached, the energy cost is expensive or the allowable range for the operation is narrow it is a further object to provide a method of isomerizing butene-1 contained in isobutylene into butene-2 with less isobutylene loss which is capable of reducing the resulting butene-1 content to such a concentration as enabling the use of isobutylene for producing butyl rubber.

## DETAILED DESCRIPTION OF THE INVENTION

The present inventors have made various studies on the isomerizing reaction of butene-1 contained in an isobutylene feed into butene-2 and have accomplished the present invention based on the finding that when an isobutylene feed containing from 0.2 to 2.0 % by weight of butene-1 is brought into contact with a catalyst containing a group VIII metal under the presence of from 0.002 to 0.008 mol of hydrogen containing from 200 to 20,000 mol ppm of carbon monoxide based on one mol of the isobutylene feed, the isobutylene loss caused by hydrogenation is substantially reduced and the concentration of butylene-1 contained in the isobutylene feed can be reduced to such a level as enabling isobutylene to be used for producing butyl rubber.

The method of isomerizing butene-1 contained in isobutylene into butene-2 according to the present invention has a feature in that a isobutylene feed containing from 0.2 to 2.0 % by weight of butene-1 is brought into contact with a catalyst containing group VIII metal under the presence of from 0.002 to 0.008 mol of hydrogen containing from 200 to 20,000 mol ppm of carbon monoxide based on one mol of the isobutylene feed.

A metod of isomerizing butene-1 contained in isobutylene into butene-2 according to the present invention will now be described more fully.

### Fluid to be Processed

A fluid to be processed used in the present invention is an isobutylene feed containing from 0.2 to 2.0 % by weight of butene-1. The isobutylene feed usable in the present invention is, specifically, a fraction recovered as isobutylene obtained by at first extracting and separating butadiene from $C_4$ hydrocarbon mixture obtained in an olefin plant, separating isobutylene from the remaining fraction by extraction with sulfuric acid or by reaction with methanol and then recovering as isobutylene, or an isobutylene fraction recovered from a butyl rubber production plant.

### Catalyst

The catalyst usable in the present invention is such a catalyst comprising group VIII metal carried on a support.

As the group VIII metal, nickel, palladium, etc. are used specifically, palladium being particularly preferred.

As the support, silica, alumina, silica-alumina, activated carbon, diatomaceous earth, active white clay, magnesia, etc. are used, alumina being particulary preferred among them.

The content of the group VIII metal, based on the support, is from 0.01 to 10% by weight and, more preferably, from 0.01 to 1.0% by weight.

Such a catalyst can be prepared, for example, by dissolving under heating palladium chloride with the addition of an aqueous solution of hydrochloric acid, stirring the same with the addition of purified water and support, evaporating and drying the aqueous solution to solidness and then applying drying.

Further, it is preferred to apply a hydrogen reduction step to the thus obtained catalyst since a catalyst of excellent catalytic activity can be obtained. The hydrogen reduction step to the catalyst is preferably conducted separately in a first stage hydrogen reduction step applied upon catalyst preparation and a second stage hydrogen reduction step applied just before using the thus prepared catalyst by way of the first stage hydrogen reduction step.

The first stage hydrogen reduction step for the preparation of the catalyst is, preferably, conducted by treating the catalyst, for example, with a hydrogen-containing gas containing about from 5 to 15% by volume of hydrogen diluted with an inert gas such as nitrogen, under the conditions at a temperature of from 150 to 300 $^{\circ}$C, under a pressure of from atmospheric pressure to 5 kg/cm$^2$G, at GHSV of from 10 to 500 hr$^{-1}$ and for a processing time of from 3 to 10 hours.

Further, the second stage hydrogen reduction step for the catalyst is, preferably, conducted, for example, by treating the catalyst applied with the first stage hydrogen reduction step as described above with a hydrogen-containing gas containing about from 2 to 8% by volume of hydrogen diluted with an inert gas such as nitrogen under the conditions at a temperature of from 30 to 70 $^{\circ}$C, under a pressure of from 1 to 20 kg/cm$^2$G, at GHSV of from 10 to 500 hr$^{-1}$ and for a processing time of from 3 to 50 hours.

## Amount of Hydrogen

In the present invention, hydrogen containing carbon monoxide is supplied to the reaction system upon isomerizing butene-1 contained in the isobutylene feed into butene-2, in which hydrogen may be present in an amount from 0.002 to 0.008 mol, more preferably, in an amount from 0.003 to 0.007 mol based on one mol of the isobutylene feed.

If the molar ratio of hydrogen to the isobutylene feed is less than 0.002 mol/mol, the isomerizing performance of butene-1 into butene-2 is poor and, if it exceeds 0.008 mol/mol, the amount of isobutane formed by the hydrogenation of isobutylene is increased to result in loss of isobutylene,

## Amount of Carbon Monoxide

In the present invention, carbon monoxide is supplied together with hydrogen to the reaction system upon isomerizing butene-1 contained in the isobutylene feed into butene-2 in which carbon monoxide may be supplied at such an amount as it is present at a concentration from 200 to 20,000 mol ppm, preferably, 500 to 20,000 mol ppm in hydrogen supplied to the reaction system. Further, in a case where butene-1 is isomerized nearly to the equlibrium composition of butene- 1 and butene-2, it is preferred to supply carbon monoxide in such an amount that the concentration in hydrogen is from 2,000 to 20,000 mol ppm.

Further, in the present invention, carbon monoxide may be previously mixed with hydrogen and then supplied to the reaction system for conducting the reaction of isomerizing butene-1 into butene-2, or carbon monoxide and hydrogen may be separately supplied to the reaction system for conducting the reaction of isomerizing butene-1 into butene-2.

In the isomerizing method according to the present invention, if the concentration of carbon monoxide in hydrogen is less than 200 mol ppm, isomerizing performance of butene-1 into butene-2 is reduced and, at the same time, the amount of isobutane formed as the hydrogenation product of isobutylene is increased, whereas the isomerizing performance of butene-1 into butene-2 is reduced if the concentration of carbon monoxide in hydrogen exceeds 20,000 mol ppm, and none of the cases is preferred.

Further, in the present invention, if the supply of carbon monoxide to the reaction system is interrupted,

4

for example, in the course of the reaction, catalyst activity directly returns to that before supplying carbon monoxide. It will be easily understood in view of the above that the isomerizing performance can not be improved even if the catalyst is used for the isomerizing reaction after pre-treatment with carbon monoxide.

Since hydrogen and carbon monoxide supplied to the reaction system can easily be separated as light fractions in a distillation column for removing butene-2 formed in the isomerizing reaction from isobutylene by distillation, they are not mixed into the isobutylene product.


Condition of Contact


The contact between the isobutylene feed and the catalyst may be conducted in a liquid phase reaction or gas phase reaction and the contact method can properly be selected from the methods known so far. For instance, there can be employed a method of bringing the isobutylene feed and the catalyst into contact in a fixed bed system, a method of bringing them into contact in a movable bed system or bringing them into contact in a fluidized bed system. Depending on the case, the isobutylene feed and the catalyst may be brought into contact batchwise.

Referring to the temperature for the isomerizing reaction, since the equilibrium of butene-1 and butene-2 at a lower temperature is more advantageous for the formation of butene-2 and the reaction at a lower temperature is more advantageous in view of energy loss, etc., it is preferred to conduct the reaction within a temperature range not requiring cooling and at a temperature as low as possible. In the present invention, the temperature for the isomerizing reaction is from 10 to 100°C and, preferably, from 30 to 70°C.

The reaction pressure in the liquid phase reaction may be greater than a pressure for keeping the isobutylene feed in the liquid phase at the reaction temperature. For instance, it is required a pressure of higher than 10 kg/cm²G at a reaction temperature of 30 to 55°C and higher than 15 kg/cm²G at a reaction temperature from 55 to 70°C.

The reaction pressure in the gas phase reaction may be about 1 to 7 kg/cm²G.

The time of contact between isobutylene feed and the catalyst in the liquid phase reaction is from 0.1 to 20 hr⁻¹, preferably, from 1 to 10 hr⁻¹ of liquid space velocity (L. H. S. V.).

The time of contact between isobutylene feed and the catalyst in the gas phase reaction is from 20 to 5,000 hr⁻¹, preferably, from 200 to 2,000 hr⁻¹ of gas space velocity (G. H. S. V.)

With the method according to the present invention, it is possible to isomerize butene-1 in the isobutylene feed into butene-2 at a high conversion ratio of higher than 80% and, at the same time, to suppress isobutylene loss caused by the conversion to isobutane by hydrogenation within 0.3% based on isobutylene supplied.

The present invention will be described below referring to examples but the invention is not restricted by these examples.

Unless otherwise specified, "%" in the examples is based on the weight.


Example 1


After adding 250 ml of 4% hydrochloric acid solution to 8.4 g of palladium chloride, they were heated to dissolve palladium chloride. 1750 ml of purified water and 1 kg of globular γ-alumina were added and stirred and, thereafter, the aqueous solution was dried to solidness by evaporation at 110°C. After drying the resultant solid at 200°C in a nitrogen gas stream, hydrogen reduction was conducted with a nitrogen gas containing 10% by volume of hydrogen for 6 hours under the following conditions: at 220°C, 3 kg/cm²G and gas space velocity of 150 hr⁻¹, to obtain a catalyst. The specific surface area of the catalyst was 120 m²/g and the packed specific gravity was 0.85 kg/liter.

After charging 35 ml of the thus prepared catalyst into a fixed bed reactor (16 mmø x 300 mm), a nitrogen gas containing 5% by volume of hydrogen was caused to pass over one day and one night under the conditions at 40°C, 3 kg/cm²G and gas space velocity (G.H.S.V.) of 150 hr⁻¹ and, further, hydrogen reduction treatment was applied.

Isobutylene feed of a composition shown in Table 1 below was passed to the hydrogen-treated catalyst, by using hydrogen containing 2,400 mol ppm of carbon monoxide, under the conditions of hydrogen isobutylene feed molar ratio of 0.006 mol/mol, reaction temperature at 40°C, pressure of 15 kg/cm²G and LHSV of 2hr⁻¹, to conduct isomerizing reaction of butene-1 contained in isobutylene into butene-2.

Table 2 shows the reaction conditions and the composition of the product in this case.

## Example 2

Isomerizing reaction was conducted in the same procedures as those in Example 1 except for using hydrogen containing 16,000 mol ppm of carbon monoxide.

The results are shown in Table 2.

## Comparative Example 1

Isomerizing reaction was conducted in the same procedures as those in Example 1 except for using hydrogen not containing carbon monoxide.

The results are shown in Table 2.

## Comparative Example 2

Isomerizing reaction was conducted in the same procedures as those in Example 1 except for using hydrogen containing 100 mol ppm of carbon monoxide.

The results are shown in Table 2.

## Comparative Example 3

Isomerizing reaction was conducted in the same procedures as those in Example 1 except for using hydrogen containing 67,000 mol ppm of carbon monoxide.

The results are shown in Table 2.

## Example 3

Isomerizing reaction was conducted in the same procedures as those in Example 1 except for changing the molar ratio of hydrogen/isobutylene feed to 0.008 mol/mol.

The results are shown in Table 2.

## Example 4

The isomerizing reaction was conducted in a gas phase in the same procedures as those in Example 1 except for using hydrogen containing 660 mol ppm of carbon monoxide, changing the molar ratio of hydrogen/isobutylene feed to 0.004 mol/mol, setting the reaction temperature to 60°C, reaction pressure to 5 kg/cm²G and GHSV to 250 hr⁻¹.

## Comparative Example 4

After charging 35 ml of the catalyst used in Example 1 after treating with the first stage hydrogen reduction into a fixed bed reactor (16 mmØ x 300 mm), carbon monoxide treatment was conducted for the catalyst by passing a nitrogen gas containing 2,000 mol ppm of carbon monoxide under the conditions at 40°C, 3 kg/cm²G and GHSV of 150 hr⁻¹ over one day and one night. Subsequently, the second stage hydrogen reduction treatment was conducted under the same conditions as those in the carbon monoxide treatment with a nitrogen gas containing 5% by volume of hydrogen.

Isomerizing reaction was conducted in the same procedures as those in Example 1 except for using the thus prepared catalyst and using hydrogen not-containing carbon monoxide.

The results are shown in Table 2.

Table 1

| Isobutylene feed | Composition | Boiling point |
|---|---|---|
| Ingredient | (%) | (°C) |
| n-butane | 0.03 | -0.5 |
| Butene-1 | 0.50 | -6.3 |
| Isobutylene | 97.87 | -6.9 |
| Trans-butene-2 | 1.10 | 0.9 |
| Cis-butene-2 | 0.48 | 3.7 |
| Butadiene-1,3 | 0.02 | -4.7 |

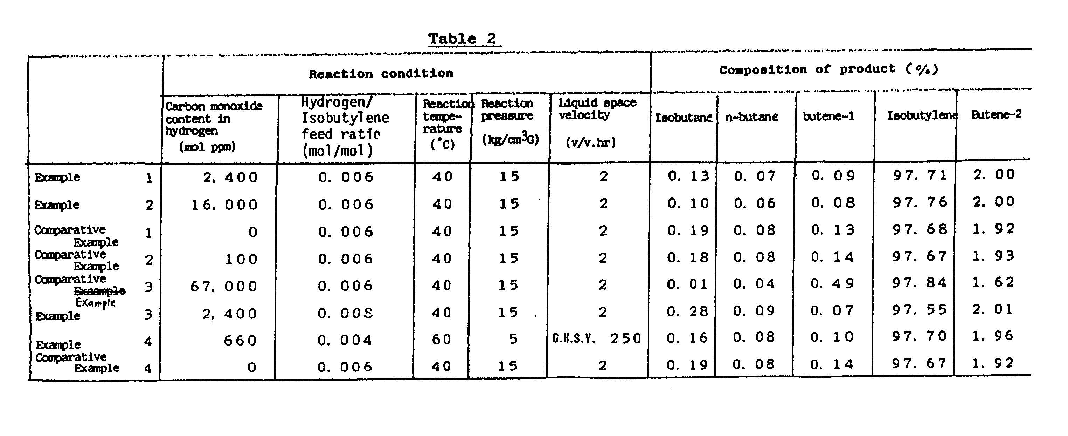

**Claims**

1. A method of isomerizing butane-1 contained in a isobutylene feed comprising isobutylene and from 0.2 to 2.0 % by weight of said butane 1 comprising:
contacting said 1 sobutylene feed with a catalyst containing a Group VIII metal and from 0.002 to 0.008 moles of hydrogen said hydrogen containing from 200 to 20,000 mol ppm of carbon monoxide based each mol of said isobutylene feed.

2. The method recited in claim 1 wherein said catalyst comprises palladium carried on a support.

3. The method recited in claim 2 wherein said support is alumina.

## Table 2

| | | Reaction condition | | | | | Composition of product (%) | | | | |
|---|---|---|---|---|---|---|---|---|---|---|---|
| | | Carbon monoxide content in hydrogen (mol ppm) | Hydrogen/ Isobutylene feed ratio (mol/mol) | Reaction tempe-rature (°C) | Reaction pressure (kg/cm³G) | Liquid space velocity (v/v.hr) | Isobutane | n-butane | butene-1 | Isobutylene | Butene-2 |
| Example | 1 | 2,400 | 0.006 | 40 | 15 | 2 | 0.13 | 0.07 | 0.09 | 97.71 | 2.00 |
| Example | 2 | 16,000 | 0.006 | 40 | 15 | 2 | 0.10 | 0.06 | 0.08 | 97.76 | 2.00 |
| Comparative Example | 1 | 0 | 0.006 | 40 | 15 | 2 | 0.19 | 0.08 | 0.13 | 97.68 | 1.92 |
| Comparative Example | 2 | 100 | 0.006 | 40 | 15 | 2 | 0.18 | 0.08 | 0.14 | 97.67 | 1.93 |
| Comparative Example | 3 | 67,000 | 0.006 | 40 | 15 | 2 | 0.01 | 0.04 | 0.49 | 97.84 | 1.62 |
| Example | 3 | 2,400 | 0.008 | 40 | 15 | 2 | 0.28 | 0.09 | 0.07 | 97.55 | 2.01 |
| Example | 4 | 660 | 0.004 | 60 | 5 | G.H.S.V. 250 | 0.16 | 0.08 | 0.10 | 97.70 | 1.96 |
| Comparative Example | 4 | 0 | 0.006 | 40 | 15 | 2 | 0.19 | 0.08 | 0.14 | 97.67 | 1.92 |

EP 0 380 374 A1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,A | US-A-3764633 (GARNER ET AL.) <br> * column 9, line 3 - column 9, line 54; claims 1, 4, 5, 8 * | 1 | C07C5/25 <br> C07C7/148 <br> C07C11/08 |
| A | US-A-3531545 (GARNER ET AL.) <br> * claims 1-3 * | 1-3 | |
| A | EP-A-288362 (INSTITUT FRANCAIS DU PETROLE) <br> * claim 1 * | 1 | |
| A | EP-A-170182 (BASF) <br> * claims 1-13 * | 1-3 | |
| D,A | FR-A-2528033 (EXXON RESEARCH) <br> * claims 1, 2, 4, 5 * <br> & JP-A-5905129 | 1-3 | |
| A | DE-A-2223513 (BP CHEMICALS) <br> * claims 1-3, 5, 6 * | 1-3 | |

**TECHNICAL FIELDS SEARCHED (Int. Cl.5)**

C07C

The present search report has been drawn up for all claims

| Place of search | Date of completion of the search | Examiner |
|---|---|---|
| BERLIN | 03 MAY 1990 | PROBERT,C |